# FASCICULE DE BREVET EUROPEEN

(11) **EP 3 338 740 B1**
(45) Date de publication et mention de la délivrance du brevet: **24.07.2019**
(21) Numéro de dépôt: 17210319.4
(22) Date de dépôt: 22.12.2017
(51) Int. Cl.: A61F 2/38

(54) **PROTHÈSE DE GENOU À PALIER FIXE**
KNIEPROTHESE MIT FESTLAGER
KNEE PROSTHESIS WITH FIXED BEARING

(30) Priorité: 23.12.2016 FR 1670780
(43) Date de publication de la demande: 27.06.2018
(73) Titulaire: X.NOV IP, 1882 Luxembourg (LU)
(72) Inventeur: BIEGUN, Frédérique, 2926 Boncourt (CH); BIEGUN, Jean-François, 2926 Boncourt (CH); MARCEAUX, Pascal, 52000 Chaumont (FR); LOEHLE, Pascal, 2904 Bressaucourt (CH)
(74) Mandataire: Eidelsberg, Olivier Nathan

(56) Documents cités:
- FR-A1- 2 568 467
- FR-A1- 2 615 726
- FR-A1- 2 987 738
- US-A1- 2016 310 284

## Description

La présente invention se rapporte à une prothèse de genou dite à palier ou insert fixe, qui comporte un composant fémoral destiné à être solidarisé au fémur et ayant un condyle médial et un condyle latéral ; un ménisque ou insert ; et un plateau tibial destiné à être solidarisé au tibia, notamment ancré au tibia par une quille d'ancrage faisant saillie de la surface inférieure du plateau, l'insert étant fixe par rapport au plateau.

On connaît déjà dans l'art antérieur des systèmes de prothèse de genou de ce genre, par exemple de US 8 128 703 ou de FR 2 615 726. La fixation, notamment pour empêcher une rotation mutuelle, du plateau et de l'insert, est réalisée par une structure complexe, difficile à fabriquer en série. En outre, ces systèmes de l'art antérieur ont tendance à user rapidement l'insert en matière polyéthylène tendre par rapport à la matière plus dure, notamment métallique, du plateau, de sorte que ces prothèses de l'art antérieur subissent une forte usure et ont une durée de vie limitée.

De FR 2 615 726, on connaît un ensemble suivant le préambule de la revendication 1.

La présente invention vise à mettre à disposition un ensemble pour une prothèse de genou, constitué d'un plateau tibial et d'un insert, en une matière plus tendre que celle du plateau tibial, qui soit simple à fabriquer et de durée de vie plus longue.

Suivant l'invention, un ensemble pour prothèse de genou est tel que défini à la revendication 1, des modes de réalisation préférés et des perfectionnements étant définis aux sous-revendications.

En prévoyant ainsi une liaison plateau-insert par l'intermédiaire d'un plot et d'une cavité correspondante en partie centrale et d'au moins deux butées postérieure et antérieure se faisant face, on obtient un système à la fois simple en terme de fabrication et qui résiste bien aux contraintes tendant à user l'insert tibial, ce qui favorise une longue durée de vie de l'ensemble et de la prothèse.

Suivant un mode de réalisation préféré de l'invention, la ou les butées du côté postérieur, respectivement antérieur, font saillie du plateau tibial au niveau du bord périphérique du plateau tibial et leur cavité respective dans l'insert tibial est constituée d'une encoche ouverte sur le dessous et sur le côté postérieur, respectivement antérieur, de l'insert.

Suivant un mode de réalisation préféré de l'invention, il est prévu deux butées antérieures et une butée postérieure, la butée postérieure ayant la forme d'un V en étant disposée sensiblement centralement par rapport aux deux butées antérieures, la butée centrale antérieure ayant deux faces planes intérieures mutuellement inclinées, chacune parallèle à une face plane intérieure de l'une des deux butées antérieures.

Suivant un mode de réalisation préféré de l'invention, une nervure, notamment périphérique, notamment circulaire, fait saillie latéralement du plot central pour permettre un encliquetage du plot dans la cavité centrale de l'insert, une rainure de réception de la nervure étant formée dans la paroi intérieure de la cavité.

La présente invention se rapporte également à une prothèse totale du genou comportant une partie fémorale et un ensemble suivant l'invention.

A titre d'exemple, on décrit maintenant un mode de réalisation préféré de l'invention en se reportant aux dessins dans lesquels :
- la figure 1: est une vue en perspective éclatée d'un ensemble constitué d'un plateau tibial et d'un insert tibial, suivant l'invention ;
- la figure 2: est une vue en perspective suivant un axe différent de l'ensemble de la figure 1 ;

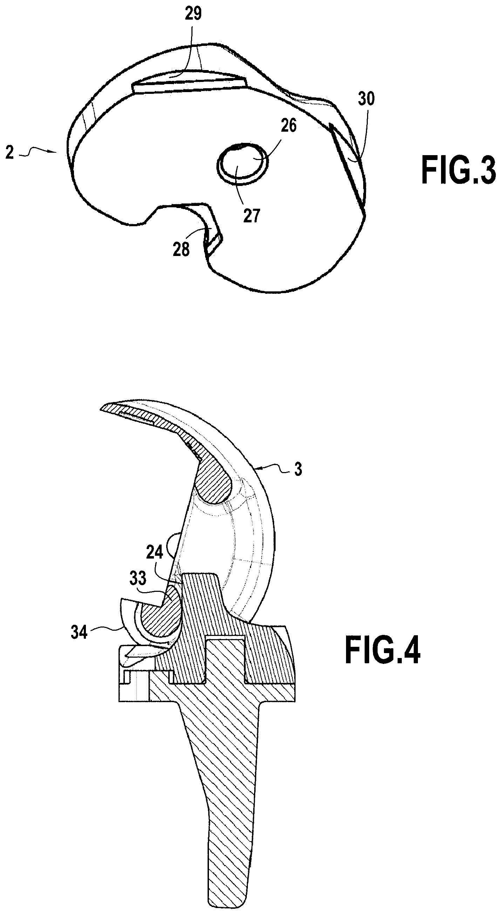

- la figure 3: est une vue en perspective de dessous de l'insert des figures 1 et 2 ; et
- la figure 4: est une vue en coupe dans le plan sagittal d'une prothèse du genou comportant l'ensemble tibial des figures 1 à 3 et une partie fémorale.

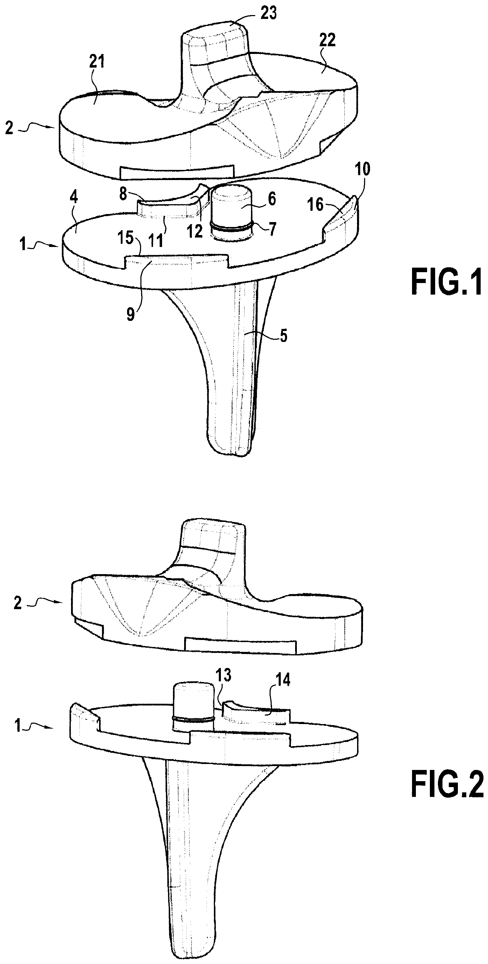

A la figure 1, il est représenté un plateau 1 tibial et un insert 2 tibial formant un mode de réalisation d'un ensemble suivant l'invention destiné à coopérer avec une partie 3 fémorale pour former une prothèse totale du genou. Le plateau est en un matériau, par exemple en métal, plus dur que le matériau de l'insert, qui est par exemple en polyéthylène.

L'ensemble suivant l'invention est de type à insert fixe, c'est-à-dire que l'insert 2, en position d'utilisation implantée, est fixe par rapport au plateau 1 tibial, et notamment ne peut pas pivoter par rapport à celui ci, notamment par rapport à l'axe mécanique du tibia.

Le plateau 1 tibial comporte une base 4 formant plateau. De sa face inférieure s'étend une quille 5 destinée à être ancrée dans le tibia du patient auquel sera implantée la prothèse. De la face supérieure, c'est-à-dire opposée à la face de laquelle s'étend la quille 5, fait saillie un plot 6 de forme cylindrique circulaire. Ce plot 6 est disposé sensiblement au milieu du plateau 4 sensiblement au-dessus de l'axe de la quille 5. Une nervure 7 périphérique fait saillie latéralement du plot 6. Cette nervure 7 a pour fonction d'assurer la fixation par encliquetage du plot 6 à l'insert 2 dans une cavité formée dans l'insert 2, qui est décrit ultérieurement.

Le bord périphérique du plateau comporte un tronçon antérieur concave (sa concavité est tournée vers l'intérieur du plateau) et du côté postérieur deux tronçons latéraux concaves séparés par un tronçon central convexe. Trois murets 8, 9 et 10 formant butées font saillie également de la face supérieure du plateau 4. Le premier muret 8 est disposé du côté postérieur sensiblement au niveau de l'axe antéro-postérieur. Le muret 8 est disposé sensiblement au niveau du tronçon central convexe du côté postérieur du bord périphérique du plateau 4. Il a une forme en V, ayant deux bras 11 et 12 gauche et droit (ou médial et latéral), la flèche du V étant orientée vers le plot 6. Les deux bras 11 et 12 ont chacun une face intérieure, respectivement 13 et 14, tournée vers l'intérieur du plateau 4, les deux faces 13 et 14 étant planes. Du côté opposé, le muret a une face concave épousant la forme du bord du plateau. Les deux faces 13 et 14 sont disposées symétriquement par rapport à l'axe antéro-postérieur.

Font saillie de la face supérieure du plateau 4 également deux murets 9 et 10, qui sont disposées symétriquement par rapport à l'axe antéro-postérieur, en étant sensiblement au niveau du bord périphérique du côté antérieur du plateau 4. Les deux murets 9 et 10 gauche et droite (ou latérale et médiale) antérieures ont une forme identique et ont chacune une face 15 et 16 respective intérieure. Les deux faces 15 et 16 sont planes. La face 15 du muret 9 gauche antérieur est parallèle à la face 13 plane du bras 11 du muret 8 postérieur, tandis que la face 16 du muret 10 droit antérieur est parallèle à la face 14 du bras 12 droit du muret 8 central postérieur. Du côté opposé, les murets ont une face concave respective épousant la forme du bord du plateau.

L'insert 2 est constitué d'un bloc. Deux surfaces concaves 21 et 22 gauche et droite (ou latérale et médiale) sont formées à la face supérieure de l'insert 2 et sont destinées à coopérer avec des condyles 34 latéral et médial de forme sensiblement sphérique de la partie 3 fémorale de la prothèse du genou d'une manière bien connue dans le domaine. De même, un plot 23 fait saillie de la face supérieure de l'insert 2 et est destiné à coopérer avec une came tibiale 33 s'étendant entre les deux condyles de la partie fémorale dans la direction médio-latérale, le roulement de la came 33 sur la face verticale 24 postérieure du plot 23 de l'insert 2 assurant la flexion relative de la partie fémorale par rapport au plateau tibial.

Dans la partie inférieure de l'insert 2 tibial sont creusées quatre cavités 26, 28, 29 et 30 destinées à coopérer respectivement avec le plot 6 et les murets 8, 9 et 10. Une cavité 26 est creusée sensiblement au milieu de l'insert tibial et est de forme complémentaire du plot 6 central issu du plateau tibial. En outre, il est creusé dans la paroi de la cavité 26 cylindrique circulaire une rainure 27 périphérique destinée à coopérer avec la nervure 7 pour assurer une fixation par encliquetage de l'insert 2 au plateau 4 et empêcher ainsi un décollement vers le haut de l'insert 2 par rapport au plateau 4. D'autre part, il est formé au niveau du bord inférieur de l'insert 2 des cavités en forme d'encoches respectivement 28, 29 et 30 de forme complémentaire respectivement des murets 8, 9 et 10. Ainsi, chaque cavité en forme d'encoches 28, 29, 30 comporte une paroi contre laquelle vient porter la face intérieure du muret qu'elle reçoit. Ainsi, l'insert 2, lorsqu'il est disposé sur le plateau 4 tibial, pénètre par le plot 6 dans la cavité 26 et par les murets 8, 9 et 10 dans les encoches 28, 29 et 30. On assure ainsi par la coopération des encoches, cavité, plot et murets la fixation relative du plateau 4 et de l'insert 2, la nervure 7 d'encliquetage assurant la fixation dans la direction verticale, tandis que la coopération des murets 8, 9 et 10 avec les encoches 28, 29 et 30 empêche toute rotation axiale de l'insert 2 par rapport au plateau 4 tibial.

Mesurée le long du bord périphérique du plateau, les deux murets antérieurs sont séparés d'une distance correspondant à l'extension du muret postérieur ou à sensiblement cette extension.

La fabrication de l'insert 2 et du plateau 4 tibial est facilitée, les formes géométriques étant simples et, en outre, la disposition relative des faces intérieures des butées 8, 9 et 10 assure une excellente résistance à l'usure lors des nombreuses flexions effectuées par le genou, et notamment par la partie 3 fémorale par rapport au plateau 4 tibial, et une grande longévité à la prothèse.

## Revendications

1. Ensemble pour prothèse de genou, comportant un plateau (4) tibial et un insert (2), le plateau tibial ayant une face supérieure, sur laquelle est disposé l'insert, et une face inférieure destinée à être solidarisée au tibia, notamment par ancrage du plateau à l'extrémité proximale ou supérieure du tibia par l'intermédiaire d'une quille (5) issue du plateau, un plot (6), notamment cylindrique, en particulier circulaire, faisant saillie de la face supérieure du plateau tibial et étant reçu dans une cavité (26) formée dans l'insert, et au moins deux butées (8, 9, 10) respectivement postérieure et antérieure faisant saillie de la face supérieure du plateau tibial, les au moins deux butées étant disposées de part et d'autre du plot dans la direction antéro-postérieure, **caractérisé en ce que** chaque butée (8, 9, 10) est reçue dans une cavité (28, 29, 30) formée dans l'insert tibial.

2. Ensemble suivant la revendication 1, **caractérisé en ce que** chaque butée a au moins une face intérieure (11, 12, 15, 16), de préférence plane, tournée vers l'intérieur du plateau, la face intérieure (11 ; 12) de la butée du côté postérieur faisant face à la face intérieure (15 ; 16) de la butée du côté antérieur.

3. Ensemble suivant la revendication 1 ou 2, **caractérisé en ce que** chaque cavité (28, 29, 30) en forme comporte une paroi respective contre laquelle vient porter la face intérieure (11, 12, 15, 16) d'un muret qu'elle reçoit.

4. Ensemble suivant la revendication 2 ou 3, **caractérisé en ce que** la face intérieure (11 ; 12) de la butée du côté postérieur est parallèle à la face intérieure (15 ; 16) de la butée du côté antérieur.

5. Ensemble suivant l'une des revendications 1 à 4, **caractérisé en ce que** la ou les butées (8, 9, 10) du côté postérieur, respectivement antérieur, font saillie du plateau tibial au niveau du bord périphérique du plateau tibial et leur cavité (28, 29, 30) respective dans l'insert tibial est constituée d'une encoche ouverte sur le dessous et sur le côté postérieur, respectivement antérieur, de l'insert.

6. Ensemble suivant l'une des revendications 1 à 5, **caractérisé en ce qu'**il est prévu deux butées (9, 10) antérieures et une butée (8) postérieure, la butée postérieure ayant la forme d'un V en étant disposée sensiblement centrale par rapport aux deux butées antérieures, la butée centrale postérieure ayant deux faces (11, 12) planes intérieures mutuellement inclinées, chacune parallèle à une face plane (15, 16) intérieure de l'une des deux butées antérieures.

7. Ensemble suivant l'une des revendications 1 à 6, **caractérisé en ce que** les deux butées antérieures sont espacées, le long du bord périphérique, d'une distance correspondant à l'extension de la butée postérieure le long du bord périphérique.

8. Ensemble suivant l'une des revendications 1 à 7, **caractérisé en ce qu'**une quille (5) fait saillie de la face inférieure du plateau (4).

9. Ensemble suivant l'une des revendications 1 à 8, **caractérisé en ce que** le plot (6) est de forme cylindrique circulaire.

10. Ensemble suivant la revendication 8 ou 9, **caractérisé en ce que** le plot est disposé sensiblement à l'opposé de la quille.

11. Ensemble suivant l'une des revendications 1 à 10, **caractérisé en ce qu'**une nervure, notamment périphérique, notamment circulaire, fait saillie latéralement du plot central pour permettre un encliquetage du plot dans la cavité centrale de l'insert, une rainure de réception de la nervure étant formée dans la paroi intérieure de la cavité.

12. Ensemble suivant l'une des revendications précédentes, **caractérisé en ce qu'**il est prévu une unique quille (5) issue du plateau (4) tibial pour la solidarisation de ce dernier au tibia.

13. Ensemble suivant l'une des revendications précédentes, **caractérisé en ce que** chaque butée (8, 9, 10) est reçue dans une cavité (28, 29, 30) respective de forme complémentaire.

14. Ensemble suivant l'une des revendications précédentes, **caractérisé en ce que** le plot (6) est reçu dans une cavité de forme complémentaire débouchant à la face inférieure de l'insert.

15. Prothèse totale du genou comportant une partie fémorale et un ensemble suivant l'une des revendications précédentes.

## Patentansprüche

1. Knieprothesenanordnung, aufweisend ein Tibiaplateau (4) und einen Einsatz (2), wobei das Tibiaplateau eine obere Fläche, auf der der Einsatz angeordnet ist, und eine untere Fläche hat, die dazu bestimmt ist, fest mit der Tibia verbunden zu werden, insbesondere durch Verankerung des Plateaus mittels eines aus dem Plateau hervorgehenden Kiels (5) an dem proximalen oder oberen Ende der Tibia, wobei ein insbesondere zylindrischer, insbesondere kreisförmiger, Stift (6) aus der oberen Fläche des Tibiaplateaus vorragt und in einem im Einsatz gebildeten Hohlraum (26) aufgenommen wird, sowie mindestens zwei, jeweils hintere und vordere Anschläge (8, 9, 10), die von der oberen Fläche des Tibiaplateaus vorragen, wobei die mindestens zwei Anschläge beiderseits des Stifts in der von vorne nach hinten gehenden Richtung angeordnet sind,
**dadurch gekennzeichnet, dass** jeder Anschlag (8, 9, 10) in einem in dem Tibia-Einsatz gebildeten Hohlraum (28, 29, 30) aufgenommen ist.

2. Anordnung nach Anspruch 1, **dadurch gekennzeichnet, dass** jeder Anschlag mindestens eine vorzugsweise ebene innere Fläche (11, 12, 15, 16) hat, die zum Inneren des Plateaus gerichtet ist, wobei die innere Fläche (11; 12) des Anschlags der hinteren Seite der inneren Fläche (15; 16) des Anschlags der vorderen Seite gegenüberliegt.

3. Anordnung nach Anspruch 1 oder 2, **dadurch gekennzeichnet, dass** jeder Hohlraum (28, 29, 30) in Form eine jeweilige Wand aufweist, an der die innere Fläche (11, 12, 15, 16) einer Trennwand anliegt, die sie aufnimmt.

4. Anordnung nach Anspruch 2 oder 3, **dadurch gekennzeichnet, dass** die innere Fläche (11; 12) des Anschlags der hinteren Seite parallel zu der inneren Fläche (15; 16) des Anschlags der vorderen Seite verläuft.

5. Anordnung nach einem der Ansprüche 1 bis 4, **dadurch gekennzeichnet, dass** der oder die Anschläge (8, 9, 10) der hinteren bzw. vorderen Seite am Umfangsrand des Tibiaplateaus vom Tibiaplateau vorragen und ihr jeweiliger Hohlraum (28, 29, 30) in dem Tibia-Einsatz an der unteren Seite und an der hinteren bzw. vorderen Seite des Einsatzes aus einer offenen Auskerbung besteht.

6. Anordnung nach einem der Ansprüche 1 bis 5, **dadurch gekennzeichnet, dass** zwei vordere Anschläge (9, 10) und ein hinterer Anschlag (8) vorgesehen sind, wobei der hintere Anschlag V-förmig ist, indem er bezüglich der beiden vorderen Anschläge im Wesentlichen mittig angeordnet ist, wobei der hintere mittlere Anschlag zwei ebene innere Flächen (11, 12) hat, die gegenseitig geneigt sind und jeweils parallel zu einer ebenen inneren Fläche (15, 16) eines der beiden vorderen Anschläge verlaufen.

7. Anordnung nach einem der Ansprüche 1 bis 6, **dadurch gekennzeichnet, dass** die beiden vorderen Anschläge entlang dem Umfangsrand mit einem Abstand beabstandet sind, der der Erstreckung des hinteren Anschlags entlang dem Umfangsrand entspricht.

8. Anordnung nach einem der Ansprüche 1 bis 7, **dadurch gekennzeichnet, dass** von der unteren Fläche des Plateaus (4) ein Kiel (5) vorragt.

9. Anordnung nach einem der Ansprüche 1 bis 8, **dadurch gekennzeichnet, dass** der Stift (6) kreisförmig zylindrisch ist.

10. Anordnung nach Anspruch 8 oder 9, **dadurch gekennzeichnet, dass** der Stift im Wesentlichen gegenüber dem Kiel angeordnet ist.

11. Anordnung nach einem der Ansprüche 1 bis 10, **dadurch gekennzeichnet, dass** eine insbesondere periphere, insbesondere kreisförmige Rippe seitlich von dem mittleren Stift vorragt, um ein Einrasten des Stifts in den mittleren Hohlraum des Einsatzes zu gestatten, wobei eine Rille zur Aufnahme der Rippe in der inneren Wand des Hohlraums ausgebildet ist.

12. Anordnung nach einem der vorhergehenden Ansprüche, **dadurch gekennzeichnet, dass** ein einziger vom Tibiaplateau (4) ausgehender Kiel (5) zu dessen fester Verbindung mit der Tibia vorgesehen ist.

13. Anordnung nach einem der vorhergehenden Ansprüche, **dadurch gekennzeichnet, dass** jeder Anschlag (8, 9, 10) in einem jeweiligen Hohlraum (28, 29, 30) mit komplementärer Form aufgenommen ist.

14. Anordnung nach einem der vorhergehenden Ansprüche, **dadurch gekennzeichnet, dass** der Stift (6) in einem Hohlraum mit komplementärer Form aufgenommen ist, der an der unteren Fläche des Einsatzes mündet.

15. Knietotalprothese, aufweisend einen Femur-Teil und eine Anordnung nach einem der vorhergehenden Ansprüche.

## Claims

1. Knee prosthesis assembly, comprising a tibial plate (4) and an insert (2), the tibial plate having an upper face, on which the insert is positioned and a lower face intended to be joined to the tibia, in particular by anchoring the plate to the proximal or upper extremity of the tibia by means of a keel (5) projecting from the plate, a post (6), essentially cylindrical, in particular circular, projecting from the upper surface of the tibial plate and received in a cavity (26) formed in the insert, and at least two posterior and anterior respectively stops (8, 9, 10) projecting from the upper surface of the tibial plate, the at least two stops being placed on each side of the post in the anterior-posterior direction, **characterised in that** each stop (8, 9, 10) is received in a cavity (28, 29, 30) formed in the tibial insert.

2. Assembly according to claim 1, **characterised in that** each stop has at least one inner face (11, 12, 15, 16), preferably flat, facing towards the inside of the plate, the inner face (11 ; 12) of the stop on the posterior side being opposite the inner face (15 ; 16) of the stop on the anterior side.

3. Assembly according to claim 1 or 2, **characterised in that** each respective cavity (28, 29, 30) has the shape of a wall against which rests the inner face (11, 12, 15, 16) of a small wall which it receives.

4. Assembly according to claim 2 or 3, characterised on that the inner face (11 ; 12) of the stop on the posterior side is parallel to the inner face (15 ; 16) of the stop on the anterior side.

5. Assembly according to one of claims 1 to 4, **characterised in that** the stop(s) (8, 9, 10) on the posterior and anterior side respectively project from the tibial plate at the surrounding edge of the tibial plate and their respective cavity (28, 29, 30) in the tibial insert consists of an open notch on the underside and on the posterior and anterior side respectively of the insert.

6. Assembly according to one of claims 1 to 5, **characterised in that** it has two anterior stops (9, 10) and one posterior stop (8), the posterior stop being in the form of a V being positioned substantially central with respect to the two anterior stops, the posterior central stop having two mutually inclined, flat lower faces (11, 12), each parallel to one inner flat face (15, 16) of one of the two anterior stops.

7. Assembly according to one of claims 1 to 6, **characterised in that** the two anterior stops are separated along the surrounding edge by a distance corresponding to the extension of the posterior stop along the surrounding edge.

8. Assembly according to one of claims 1 to 7, **characterised in that** a keel (5) projects from the lower face of the plate (4).

9. Assembly according to one of claims 1 to 8, **characterised in that** the post (6) is of a cylindrical, circular shape.

10. Assembly according to claim 8 or 9, **characterised in that** the post is positioned substantially opposite the keel.

11. Assembly according to one of claims 1 to 10, **characterised in that** a rib, in particular peripheral, in particular circular, projects laterally from the central post to permit the post to click into the central cavity of the insert, a receiving groove for the rib being formed in the inner wall of the cavity,

12. Assembly according to one of the foregoing claims, **characterised in that** it is provided with a single keel (5) projecting from the tibial plate (4) to connect the latter to the tibia.

13. Assembly according to one of the foregoing claims, **characterised in that** each stop (8, 9, 10) is received in a respective cavity (28, 29, 30) of a complementary shape.

14. Assembly according to one of the foregoing claims, **characterised in that** the post (6) is received in a cavity of a complementary shape opening onto the lower face of the insert.

15. Total knee prosthesis comprising a femoral part and an assembly according to one of the foregoing claims.
